(19) Europäisches Patentamt
European Patent Office
Office européen des brevets

(11) Veröffentlichungsnummer: **0 143 977 B1**

# (12) EUROPÄISCHE PATENTSCHRIFT

(45) Veröffentlichungstag der Patentschrift: **15.03.95**

(51) Int. Cl.6: **A61K 31/365**

(21) Anmeldenummer: **84112902.6**

(22) Anmeldetag: **25.10.84**

## (54) Bilobalid zur Anwendung als Therapeutischer Wirkstoff.

(30) Priorität: **27.10.83 DE 3338995**

(43) Veröffentlichungstag der Anmeldung:
**12.06.85 Patentblatt 85/24**

(45) Bekanntmachung des Hinweises auf die Patenterteilung:
**15.03.95 Patentblatt 95/11**

(84) Benannte Vertragsstaaten:
**AT BE CH DE FR GB IT LI LU NL**

(56) Entgegenhaltungen:

**HAGERS HANDBUCH DER PHARMAZEUTISCHEN PRAXIS, 4. Neuausgabe, Band 4, Seiten 1134-1136, 1973, Springer Verlag, Berlin, DE; P.H. LIST & L. HÖRHAMMER**

**THERAPIEWOCHE, DIAGNOSTISCHE UND THERAPEUTISCHE MITTEILUNGEN AUS DER PRAXIS, Band, 30, Nr. 36, 1980, Seiten 5852-5854, Verlag G. BRAUN, Karlsruhe, DE; F.W. KOEPPEL: "Über die Wirkung von Tebonin beim Hörverlust unbekannter Ursache und toxischer Innenohrschwerhörigkeit"**

(73) Patentinhaber: **Dr. Willmar Schwabe GmbH & Co.**
**Postfach 41 09 25**
**D-76209 Karlsruhe (DE)**

(72) Erfinder: **Chatterjee, Shyam Sunter, Dr.**
**Stettiner Strasse 1**
**D-7500 Karlsruhe 1 (DE)**
Erfinder: **Gabard, Bernard Louis, Dr.**
**Gürrichstrasse 44**
**D-7500 Karlsruhe 31 (DE)**
Erfinder: **Jaggy, Hermann E. W., Dr.**
**Kapellenweg 7**
**D-7525 Bad Schönborn 1 (DE)**

(74) Vertreter: **VOSSIUS & PARTNER**
**Postfach 86 07 67**
**D-81634 München (DE)**

EP 0 143 977 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIEBIGS ANNALEN DER CHEMIE, Band 759, 1972, Seiten 158-172; K. WEINGES et al.: "Naturstoffe aus Arzneipflanzen, XVI - NMR- und massenspektrometrischer Vergleich des Bilobalids C15H18O8 mit den Ginkgoliden C20H24O9-11

ÄRZTEZEITSCHRIFT FÜR NATURHEILVERFAHREN, Band 22, Nr. 11, November 1981, Seiten 593-603; S.S. CHATTERJEE et al.: "Neue Ergebnisse aus der Ginkgo-Forschung"

ISRAEL PHARMACEUTICAL JOURNAL, HAROKEACH HAIVRI, Band 18, Nr. 7, 1975, Seiten 337-330; P. MESNARD: "Médicaments veinotoniques á principes actifs terpéniques - sur trois acquisitions récentes

PLANTES MEDICINALES ET PHYTOTHERAPIE, Band 11, 1977, Seiten 189-197; R. ANTON: "Ginkgo et maladies vasculaires"

## Beschreibung

Die Isolierung einer Lactonverbindung mit der Summenformel $C_{15}H_{18}O_8$ aus den Blättern von Ginkgo biloba wurde 1967 von R.T. Major zuerst erwähnt (Science 157 (1967), 1270 bis 1273). Von K. Weinges und W. Bähr (Liebigs Ann. Chem. 724 (1969), 214 bis 216) wurden physikalisch-chemische Eigenschaften dieser Verbindung und Derivate beschrieben, sowie der Name Bilobalid vorgeschlagen. In einer gemeinsamen Publikation der Arbeitskreise K. Nakanishi et al., R.T. Major et al. und K. Weinges et al. (J. Amer. Chem. Soc. 93 (1971), 3544 bis 3546) wurde für Bilobalid folgende Strukturformel I vorgeschlagen

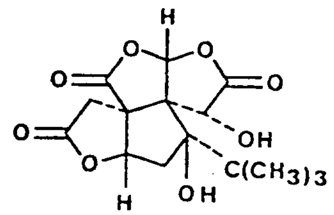

(I)

Für Bilobalid sind keine Wirkungen als Arzneistoff bekannt geworden. Doch erwähnt Mesnard, Israel Pharmaceutical Journal Bd. 18, (1975), Seiten 337-330 "ein anderes von Weinges und Bähr beschriebenes Sesquiterpen, das Bilobalid". In der Zusammenfassung dieser Arbeit werden die Wirkungen von Ginkgo biloba Extrakten Sesquiterpenen und Diterpenen zugeschrieben, ohne daß jedoch pharmakologische Untersuchungen genannt werden. In einer später erschienen Arbeit von Anton, Plantes médicinales et phytothérapie, Bd. 11 (1977), Seiten 189-197, wird berichtet, daß der Mangel an Wissen über die chemischen und pharmakologischen Zuammenhänge keine exakte Kenntnis des pharmakologisch aktiven Prinzips zuläßt. Diese Aussage wird in der Arbeit mehrfach bekräftigt und darüberhinaus in Frage gestellt, ob für Wirkungen tatsächlich ursprünglich enthaltene Verbindungen verantwortlich sind.

Es ist aber bekannt, daß Bilobalid nicht fungizid wirkt gegen Monilia fructicola und Penicillium glaucum sowie nicht bakterizid wirkt gegen Escherichia coli. Es ist ferner bekannt, daß Bilobalid nicht entzündlich wirkt am Mäuseohr.

Die aus Ginkgo biloba hergestellten bekannten Extrakte, die in der Medizin seit 1965 zur Behandlung zerebraler und peripherer arterieller Durchblutungsstörungen eingesetzt werden, enthalten als Hauptbestandteil Flavonglykoside. Ein typischer Vertreter dieser Gruppe ist das 5,7,3',4'-Tetrahydroxy-flavono-3-O-α-rhamnopyranosyl-4''-O-β-D-(6'''-trans-coumaroyl)-glucopyranosid der Formel II

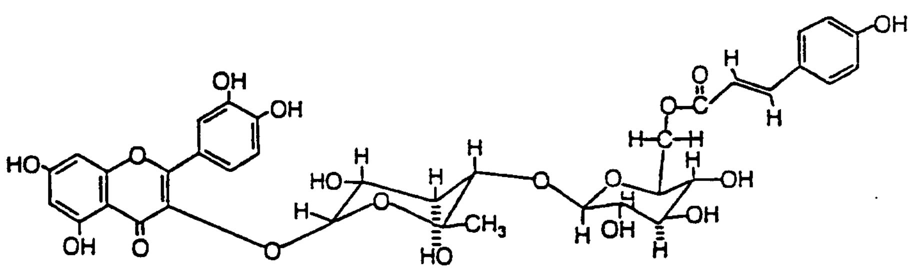

(II)

Diese Extrakte, die auch als Monoextrakte bezeichnet werden (vgl. S.S. Chatterjee und G. Trunzler, Ärztezeitschrift für Naturheilverfahren 22 (1981), 593 bis 604), können je nach der Art ihrer Herstellung noch geringe Mengen an Bilobalid und Ginkgolidenenthalten, für die aber bis jetzt keine therapeutisch brauchbaren biologischen Wirkungen bekannt waren.

Zahlreich vorliegende Berichte aus der Klinik und Praxis lassen vermuten, daß zusätzlich zu der Therapie von Durchblutungsstörungen vorhandene Neuropathien, neurologische und psychische Funktionen mit auf bestimmte Weise hergestellten Monoextrakten günstig beeinflußt werden. Bis jetzt wurden diese Wirkungen ausschließlich auf die nachgewiesenen durchblutungsfördernden Wirkungen des Monoextraktes zurückgeführt. Pharmakologische Untersuchungen haben aber gezeigt, daß der Monoextrakt zusätzliche Eigenschaften besitzt, die über die durchblutungsfördernden oder vaskulären hinausgehen und die für die in der Klinik beobachteten Besserungen der nervalen Erkrankungen verantwortlich sein könnten.

Die vorliegende Erfindung beruht auf dem überraschenden Befund, daß Bilobalid für die Behandlung von bestimmten nervalen Erkrankungen geeignet ist. Der Erfindung liegt somit die Aufgabe zugrunde, Bilobalid enthaltende Arzneimittel zur Behandlung von nervalen Erkrankungen zur Verfügung zu stellen.

Die Krankheiten, die man mit Bilobalid oder mit Bilobalid enthaltenden Arzneimitteln therapieren kann, werden als Neuropathien, Encephalopathien und Myelopathien bezeichnet und sind mindestens von einem der folgenden Symptome begleitet: Parästhesien, Paresen, Reflexanomalien, Muskelatrophien, Muskelkrämpfe,Tremor, Störungen der Oberflächen- und Tiefensensibilität, Kopf- und Gliederschmerzen, Sprach-, Seh- und Hörstörungen, Schwindel, Bewußtseinsstörungen, Koordinations- und Konzentrationsschwäche, Gedächtnisstörungen und Desorientiertheit. Die bilobalidhaltigen Medikamente eignen sich für neurologische Krankheiten, die von pathologischen Änderungen in der Myelinschicht der Nervenfasern verursacht oder davon begleitet sind.

Die große Bedeutung der Integrität des Myelins für die normalen neurologischen Funktionen ist allgemein bekannt. Sie hängt ab von einer normalen Funktionsfähigkeit der Schwann'schen Scheiden im peripheren Nervensystem und der Oligodendrozyten im Zentralnervensystem und ist außerdem nur gewährleistet, wenn die Neuronen und Axone intakt sind. Myelinschäden sind häufige Folge einer Vielzahl von nicht miteinander zusammenhängenden pathologischen Zuständen und werden ausgelöst z. B. durch Infekte, Intoxikationen, immunologische Störungen, genetische Defekte, Tumoren, Hirnödeme, Traumata und Anoxie. Myelinschäden können direkt oder indirekt auftreten. Demzufolge unterscheidet man zwischen primären und sekundären Formen. Zu den primären gehören die entzündlichen und immunologischen demyelinisierenden Erkrankungen (z.B. Multiple Sklerose, postinfektiöse Encephalitiden und das Guillain-Barré-Syndrom), die genetisch verursachten metabolischen Neuropathien (z.B. die Leukodystrophien, das Refsum-Syndrom und die Pelizaeus-Merzbacher-Krankheit) und die toxisch bedingten metabolischen Neuropathien (z.B. die diphtherische Polyneuritis und die Blei-Encephalitiden). Zu den sekundären Formen zählen die traumatischen Neuropathien (z.B. die Waller'sche Degeneration), die sklerosierenden Neuropathien (z.B. die sklerosierende Panencephalitis , die diabetischen, alkoholbedingten, serogenetischen, hereditären und vaskulären Polyneuropathien und die Myelinschäden bei Hemiplegie infolge Apoplexie.

Die komplexe Ätiologie und Pathogenese der nervalen Erkrankungen erschweren die Auswahl der pharmakologischen Modelle, die für die Prüfung von potentiell wirksamen Substanzen geeignet sind. Eine Möglichkeit ist, die therapeutische Wirkung solcher Substanzen an Tieren zu prüfen, die unter neurologischen Symptomen leiden. Die krankheitsähnlichen morphologischen, pathologischen und elektrophysiologischen Symptome kann man bei Tieren durch neurotoxische Gifte wie z.B. organische Zinnverbindungen oder Hexachlorophen [2,2'-methylenbis-(3,4,6-trichlorphenol)] hervorrufen. Es ist bekannt, daß z.B. Triäthylzinnchlorid eine Zunahme des Wassergehaltes im Gehirn von verschiedenen Spezies bewirkt und eine spezifische Myelopathie in den Nerven hervorruft; die zu Neuropathien führt (Int. Rev.Neurobiol, 12 (1970), 45 - 86). In ähnlicher Weise produziert Hexachlorophen ödematöse Schädigungen im Gehirn von vielen Spezies inklusive Menschen (Arch. Environ. Health, 23 (1971), 114 - 118; J. Pediatrics, 82 (1973), 976-981) verbunden mit Myelinverlusten und -änderungen in den Nerven (J. Lipid Res., 12 (1971), 570 - 579; Acta Neuropathol., 53 (1983) 65 - 74. Deswegen eignen sich die mit diesem Gift behandelten Tiere als Modell für die vorstehend erwähnten Krankheiten besonders gut. Eine Vergiftung mit Triäthylzinnchlorid wurde als myelinschädigendes Modell verwendet (Neurochem., 21 (1973), 357-372) und als Modell für degenerative Krankheiten neuronalen Ursprungs dargestellt (Pharmacol.Biochem. Behavior, 5 (1976), 299-307).

Die Wirksamkeit von Bilobalid wurde in folgenden pharmakologischen Modellen nachgewiesen:

Versuch 1

Männlichen Sprague-Dawley Ratten (200 - 300 g Körpergewicht) wird 14 Tage als Trinkflüssigkeit 0,002 prozentige Triäthylzinnchlorid enthaltende Wasserlösung (abgekürzt TÄZ) angeboten. Kontrolltiere erhalten Leitungswasser. Der Gingko biloba Monoextrakt (EGB) bzw. die verschiedenen Testsubstanzen werden per os 1 x täglich während dieser 14 Tage appliziert. Am 15 Tag werden die Tiere getötet, das Gehirn wird entnommen und dessen Wasser- und Elektrolytgehalt bestimmt. Das Körpergewicht der Tiere wird während des Versuchs täglich kontrolliert. Die Behandlung mit EGB, Bilobalid und/oder Bilobalid-haltigen Extrakten zeigt eine gute protektive Wirkung gegen die Abnahme des Körpergewichtes und die Zunahme des Wasser- und $Na^+$-Gehaltes im Gehirn. Die Ergebnisse sind in Tabelle I zusammengefaßt.

## Tabelle I

| Testsubstanz | Dosis | Trinkflüssigkeit | mittleres Körpergewicht (g) Anfang | mittleres Körpergewicht (g) 14 Tage | Gehirn % Wasser | Gehirn $Na^+$ mmol/kg Trockenmasse | Gehirn $K^+$ mmol/kg Trockenmasse | Gehirn $Na^+$ / $K^+$ |
|---|---|---|---|---|---|---|---|---|
| Wasser | | Wasser | 250 | 320 | 77,90 ± 0,07 | 193,6 ± 0,7 | 442,7 ± 3,9 | 0,44 ± 0,003 |
| Wasser | | TAZ 0,002 % | 250 | 204 | 80,51 ± 0,19 | 284,4 ± 7,4 | 444,4 ± 3,7 | 0,64 ± 0,020 |
| EGB | 100 mg/kg | TAZ 0,002 % | 238 | 290 | 78,36 ± 0,16 | 189,9 ± 1,7 | 442,2 ± 3,0 | 0,43 ± 0,006 |
| EGB ohne Flavone | 60 mg/kg | TAZ 0,002 % | 242 | 288 | 78,06 ± 0,15 | 186,7 ± 3,1 | 419,1 ± 2,9 | 0,44 ± 0,005 |
| Flavone aus EGB | 40 mg/kg | TAZ 0,002 % | 252 | 195 | 80,06 ± 0,27 | 241,3 ± 8,9 | 440,6 ± 3,0 | 0,55 ± 0,02 |
| Bilobalid | 20 mg/kg | TAZ 0,002 % | 289 | 325 | 77,75 ± 0,11 | 187,9 ± 10,4 | 400,6 ± 5,3 | 0,45 ± 0,05 |
| | 10 mg/kg | TAZ 0,002 % | 272 | 319 | 78,16 ± 0,09 | 198,3 ± 2,74 | 452,7 ± 12,9 | 0,44 ± 0,04 |
| | 5 mg/kg | TAZ 0,002 % | 288 | 323 | 78,57 ± 0,19 | 162,6 ± 5,4 | 418,9 ± 14,4 | 0,39 ± 0,020 |
| EGB ohne Flavone und ohne Bilobalid | 60 mg/kg | TAZ 0,002 % | 245 | 210 | 80,29 ± 0,11 | 297,8 ± 6,7 | 429,4 ± 3,3 | 0,69 ± 0,01 |

Versuch 2

In einem weiteren Experiment werden männliche Sprague-Dawley Ratten (200 - 250 g Körpergewicht) zwei Tage mit 20 mg/kg Hexachlorophen (in 0,2 % Agar suspendiert) und am 3. Tag mit nur 10 mg/kg intraperitoneal gespritzt. Die Testsubstanzen werden nach der Verabreichung von Hexachlorophen per os

gegeben. Die Kontrolltiere erhalten nur 0,2prozentigen Agar i.p. bzw. Leitungswasser per os. Am 4. Tag werden die Tiere getötet und der Wasser und Elektrolytgehalt im Gehirn wird bestimmt. Das Körpergewicht der Tiere wird während der 4 Tage täglich gemessen. Wie die Ergebnisse in der Tabelle II zeigen, schützt die Behandlung mit EGB, Bilobalid und/oder Bilobalid-haltigen Extrakten die Tiere gegen die Entwicklung eines Gehirnödems und verhindert die Körpergewichtsabnahme.

Tabelle II

| Testsubstanz | | Intraperitoneale Behandlung | mittleres Körpergewicht (g) | | Gehirn | | | |
|---|---|---|---|---|---|---|---|---|
| | Dosis | | Anfang | 4. Tag | % Wasser | $Na^+$ | $K^+$ | $Na^+$ / $K^+$ |
| | | | | | | mmol/kg Trockenmasse | | |
| Wasser | | 0,2 %ig Agar | 238 | 266 | 78,50 ± 0,14 | 207,6 ± 4,0 | 433,6 ± 4,1 | 0,48 ± 0,009 |
| Wasser | | Hexachlorophen | 237 | 228 | 80,01 ± 0,19 | 265,4 ± 2,6 | 423,6 ± 2,9 | 0,63 ± 0,02 |
| EGB | 100 mg/kg | Hexachlorophen | 245 | 253 | 79,10 ± 0,21 | 240,4 ± 10,9 | 432,3 ± 1,6 | 0,56 ± 0,02 |
| | 50 mg/kg | Hexachlorophen | 236 | 243 | 79,40 ± 0,17 | 240,7 ± 13,1 | 421,0 ± 2,6 | 0,57 ± 0,03 |
| | 25 mg/kg | Hexachlorophen | 230 | 236 | 79,86 ± 0,12 | 245,9 ± 11,3 | 417,8 ± 2,6 | 0,59 ± 0,02 |
| EGB ohne Flavone | 60 mg/kg | Hexachlorophen | 242 | 252 | 78,74 ± 0,24 | 238,2 ± 12,8 | 437,9 ± 2,0 | 0,54 ± 0,03 |
| | 30 mg/kg | Hexachlorophen | 230 | 237 | 79,27 ± 0,09 | 227,7 ± 17,2 | 423,4 ± 4,0 | 0,54 ± 0,02 |
| | 15 mg/kg | Hexachlorophen | 239 | 240 | 79,55 ± 0,30 | 275,6 ± 10,3 | 422,6 ± 4,9 | 0,65 ± 0,02 |
| Bilobalid | 5 mg/kg | Hexachlorophen | 264 | 270 | 77,93 ± 0,17 | 187,9 ± 4,0 | 414,5 ± 4,4 | 0,45 ± 0,01 |
| Flavone aus EGB | 40 mg/kg | Hexachlorophen | 250 | 240 | 80,51 ± 0,23 | 291,4 ± 20,0 | 432,7 ± 5,4 | 0,67 ± 0,04 |

Versuch 3

Die kurative Wirkung vom EGB wird in einem dritten Experiment nachgewiesen. Den Tieren wird wie im Versuch 1 als Trinkflüssigkeit während 14 Tage eine 0,002prozentige Triäthylzinnchloridlösung anstatt Trinkwasser angeboten. Die Behandlung mit den Testsubstanzen wird am 15. Tag mit 1 x täglich per os 5 Tage/Woche angefangen unter gleichzeitigem Ersatz der TÄZ-Lösung durch normales Leitungswasser. Der Wasser- und Elektrolytgehalt des Gehirns wird an verschiedenen Zeitpunkten nach Entzug der TÄZ-Lösung bzw. nach Beginn der Behandlung gemessen. Die Behandlung mit EGB bewirkt eine schnellere Rückkehr des Wasser- und $Na^+$-Gehaltes des Gehirns zu den Normwerten und zeigt somit, daß eine gute kurative Wirkung vorhanden ist. Diese Versuchsergebnisse sind in den Abbildungen 1 und 2 dargestellt.

Versuch 4

Die Untersuchungen der schützenden Wirkung gegen die Myelinschädigungen bzw. ihren Ausdruck als Neuropathien erfolgt ebenfalls an dem Triäthylzinnchlorid-Modell. Vergiftet man die Tiere nicht 14 Tage, sondern nur 6 Tage lang mit einer 0,002prozentigen TÄZ-Lösung, welche anstelle des Trinkwassers gegeben wird, so zeigen sie verschiedene Symptome einer Neuropathie, obwohl in dieser Zeit noch kein Gehirnödem feststellbar ist. Die Neuropathie wird durch Messung des Futter- und Wasserverbrauchs sowie durch Veränderungen im Körpergewicht quantifiziert. Als spezifischer Parameter wird zusätzlich die Schmerzreaktionszeit in einem Wärmeplatten-Test (bei 50 °C) gemessen ( J. Pharm. Pharmacol., 9 (1957), 381. Eine während dieser 6 Tage durchgeführte perorale Behandlung mit EGB oder Bilobalid zeigt gute protektive Wirkungen gegen die neuropathischen Symptome, wie aus Tabelle III hervorgeht.

Tabelle III

| Testsubstanz | | Trinkflüssigkeit | mittleres Körpergewicht (g) | | Futterverbrauch (g/Tier/Tag) | | Wasserverbrauch (ml/Tier/Tag) | | Wärmeplatte-Reaktionszeit (sec) | |
|---|---|---|---|---|---|---|---|---|---|---|
| | Dosis | | Anfang | Ende | Anfang | Ende | Anfang | Ende | Anfang | Ende |
| Wasser | | Wasser | 245 | 280 | 25,6 | 24,4 | 37,0 | 33,2 | 39,4 ± 3,4 | 18,6 ± 2,1 |
| Wasser | | TÄZ 0,002 % | 240 | 226 | 24,0 | 12,4 | 34,4 | 11,9 | 36,6 ± 4,3 | > 60 |
| EGB | 100 mg/kg/Tag | TÄZ 0,002 % | 245 | 257 | 25,9 | 20,2 | 37,2 | 20,7 | 36,9 ± 2,0 | 21,5 ± 3,32 |
| Bilobalid | 10 mg/kg/Tag | TÄZ 0,002 % | 241 | 261 | 24,3 | 22,1 | 35,1 | 21,9 | 28,9 ± 8,3 | 17,9 ± 5,54 |

Versuch 5

In ähnlicher Weise werden die kurativen Wirkungen von Bilobalid oder Bilobalid-haltigem Extrakt untersucht. Den Tieren wird 6 Tage lang eine 0,002prozentige TÄZ-Lösung anstatt Trinkwasser angeboten. Anschließend (ab dem 7. Tag) wird die TÄZ-Lösung durch Leitungswasser ersetzt und gleichzeitig mit der täglichen peroralen Behandlung begonnen. Es wird 6 Tage lang behandelt. Die gemessenen Parameter (Körpergewicht, Futter- und Wasserverbrauch, Wärmeplattenreaktionszeit) werden am 6. Tag vor der Behandlung und am 14. Tag nach der Behandlung ermittelt. Die Ergebnisse sind in Tabelle IV zusammengefaßt.

Tabelle IV

| Behandlung | Dosis | Körpergewicht (g) | | Futterverbrauch (g/Tier/Tag) | | Wasserverbrauch (ml/Tier/Tag) | | Wärmeplatte-Reaktionszeit (sec) | |
|---|---|---|---|---|---|---|---|---|---|
| | | nach Vergiftung | nach Behandlung | nach Vergiftung | nach Behandlung | nach Vergiftung | nach Behandlung | nach Vergiftung | nach Behandlung |
| Wasser | | 229 | 222 | 10,7 | 18,3 | 11,1 | 13 | 88,4 ± 4,7 | > 90 |
| EGB | 100 mg/kg | 212 | 260 | 11,4 | 19,7 | 10,8 | 21,7 | 84,4 ± 13 | 32,9 ± 14,9 |
| EGB | 50 mg/kg | 229 | 246 | 14,9 | 18,6 | 11,6 | 22,4 | 82,1 ± 15,5 | 48,2 ± 22,3 |
| Bilobalid | 10 mg/kg | 227 | 232 | 8,9 | 18,6 | 12,7 | 23,1 | 87,36± 7,46 | 48,58± 18,44 |
| Bilobalid | 5 mg/kg | 236 | 242 | 10,4 | 17,4 | 11,4 | 31,4 | 88,1 ± 5,67 | 61,58± 21,86 |

Die Tatsache, daß eine Behandlung mit Bilobalid enthaltendem EGB oder Bilobalid das Auftreten von neurotoxischen Symptomen und von Gehirnödem verhindern und daß schon vorhandene Schädigungen schneller rückgängig gemacht werden können, zeigt, daß diese Behandlung Myelinschädigungen und die daher entstehenden Symptome beseitigen kann.

Bilobalid kann in Form von üblichen Arzneimitteln, z.B. Salben, Lösungen, Dragees, Tabletten, Kapseln, Injektions- oder Infusionslösungen, oral oder parenteral, z.B. intramuskulär oder intravenös, oder topisch, z.B. in Form von perkutan wirkenden Pflastern, gegeben werden. Die Dosis hängt ab von der Schwere der Erkrankung und dem Gewicht des Patienten. Dragees können morgens und abends nach den Mahlzeiten gegeben werden. Als Tagesdosen werden bei den normalen Arzneiformen 5 bis 40 mg Bilobalid gegeben, bei parenteraler Applikation 0,5 bis 5 mg Bilobalid und bei kutaner Anwendung von 5 bis 100 mg Bilobalid.

Bilobalid kann z.B. nach der von K. Weinges und W. Bähr, Justus Liebigs Ann. Chem., 724 (1969), 214 - 216, angegebenen Methode aus den Blättern von Ginkgo biloba isoliert werden.

Zur Herstellung von Bilobalid enthaltenden Arzneimitteln können die üblichen Träger- und Zusatzstoffe verwendet werden. Übliche Trägerstoffe sind beispielsweise Wasser, physiologische Kochsalzlösung, Alkohole, Polyäthylenglykole, Glycerinester, Gelatine, Kohlenhydrate wie Lactose und Stärke, Magnesiumstearat, Talcum. Übliche Zusatzstoffe sind beispielsweise Konservierungsmittel, Sterilisierungsmittel, Gleitmittel, Netzmittel und Emulgatoren, Farbstoffe, Geschmackskorrigentien und Aromastoffe. Die Auswahl der Träger- und Zusatzstoffe hängt davon ab, ob die erfindungsgemäßen Zubereitungen enteral, parenteral oder lokal appliziert werden sollen.

1. Tabletten mit reinem Bilobalid

Zur Herstellung von Tabletten mit jeweils 100 mg Einzelgewicht, die 5 mg Bilobalid enthalten, benötigt man

5 g Bilobalid
58,5 g Lactose
18 g mikrokristalline Cellulose
18 g Maisstärke
0,5 g Magnesiumstearat

Die ersten vier Bestandteile werden gemischt, granuliert und nach Zugabe von Magnesiumstearat auf einer Tablettiermaschine zu Tabletten verpreßt.

2. Kapseln

7 g Bilobalid
75 g Lactose
20 g Maisstärke

Die Bestandteile werden homogen gemischt und in üblicher Weise zu Kapseln mit einem Füllgewicht von 100 mg verarbeitet.

3. Injektionsampullen

Zur Herstellung von Injektionsampullen mit jeweils 2 ml Inhalt, die 0,5 mg Bilobalid enthalten, benötigt man

0,25 g Bilobalid
9 g Natriumchlorid
ad 100 g Aqua bidest

Die ersten beiden Bestandteile werden unter leichtem Erwärmen und Rühren in Wasser gelöst. Die Lösung wird steril filtriert und in 2 ml Ampullen abgefüllt.

4. Flüssige orale Arzneiform

5 g Bilobalid
10 g Aromaessenz
5 g Natriumsaccharinat
400 g Äthylalkohol
580 g aqua dest. oder vollentsalztes Wasser.

Die ersten drei Bestandteile werden im Gemisch aus Äthanol und Wasser gelöst. Die erhaltene Lösung wird in 100 ml Fläschchen abgefüllt. Die Einzeldosis beträgt 1 ml.

5. Salbe

0,5 g Bilobalid
30 g Emulgierender Cetylstearylalkohol
35 g Dickflüssiges Paraffin
34,5 g weiße Vaseline.

Das Gemisch aus Cetylstearylalkohol, weiße Vaseline und dickflüssigem Paraffin wird geschmolzen. Sodann wird das Bilobalid eingerührt. Die Dosierung beträgt 1 bis 10 g Salbe pro Behandlung.

## Patentansprüche

1. Bilobalid zur Anwendung als nervalen Erkrankungen entgegenwirkender Stoff.

2. Bilobalid zur Anwendung gemäß Ansprüch 1 als demyelinisierenden Neuropathien, Encephalopathien, Myelopathien und Hirnödemen entgegenwirkender Stoff.

## Claims

1. Bilobalid for use as substance counteracting nervous diseases.

2. Bilobalid for use according to claim 1 as substance counteracting demyelinating neuropathies, encephalopathies, myelopathies and cerebral oedemas.

## Revendications

1. Bilobalide mis en oeuvre comme substance active contre des maladies nerveuses.

2. Bilobalide mis en oeuvre suivant la revendication 1 comme substance active contre des neuropathies démyélinisantes, des encéphalopathies, des myélopathies et des oedèmes cérébraux.

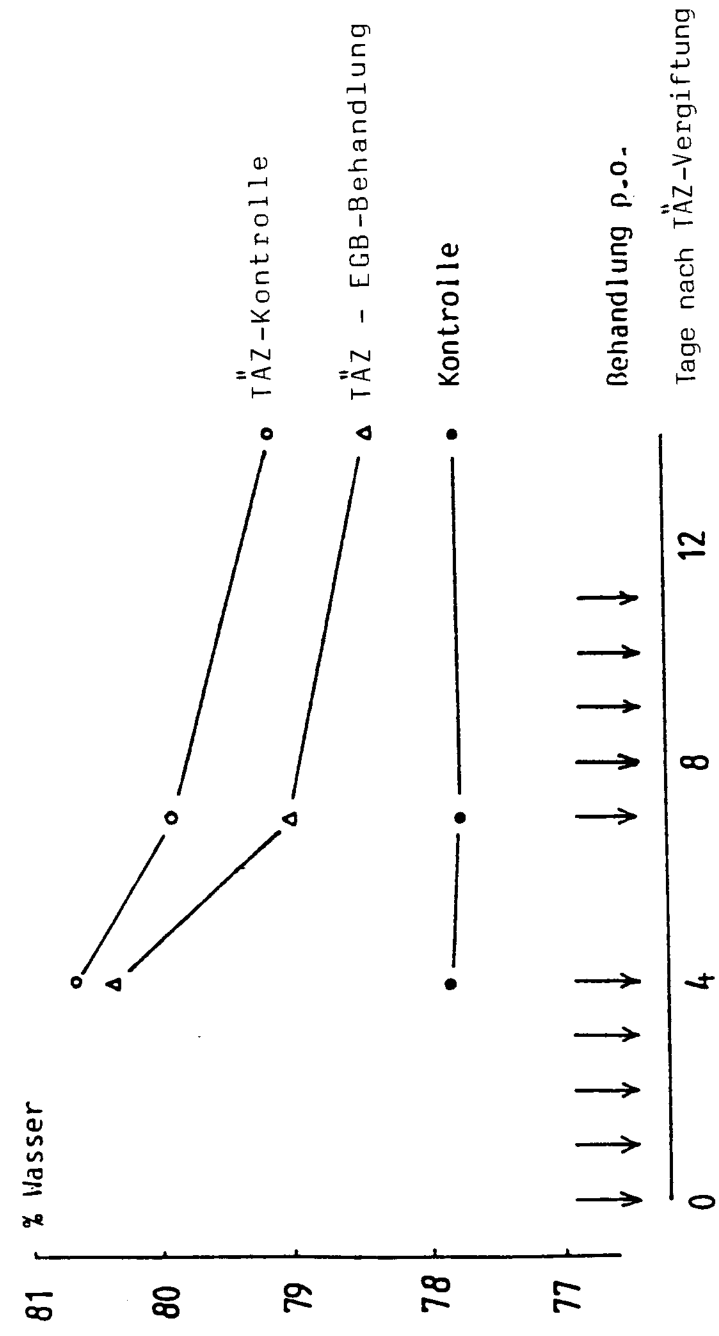
% Wasser
81
80
79
78
77
0
4
8
12
TÄZ-Kontrolle
TÄZ - EGB-Behandlung
Kontrolle
Behandlung p.o.
Tage nach TÄZ-Vergiftung

Abb. 1

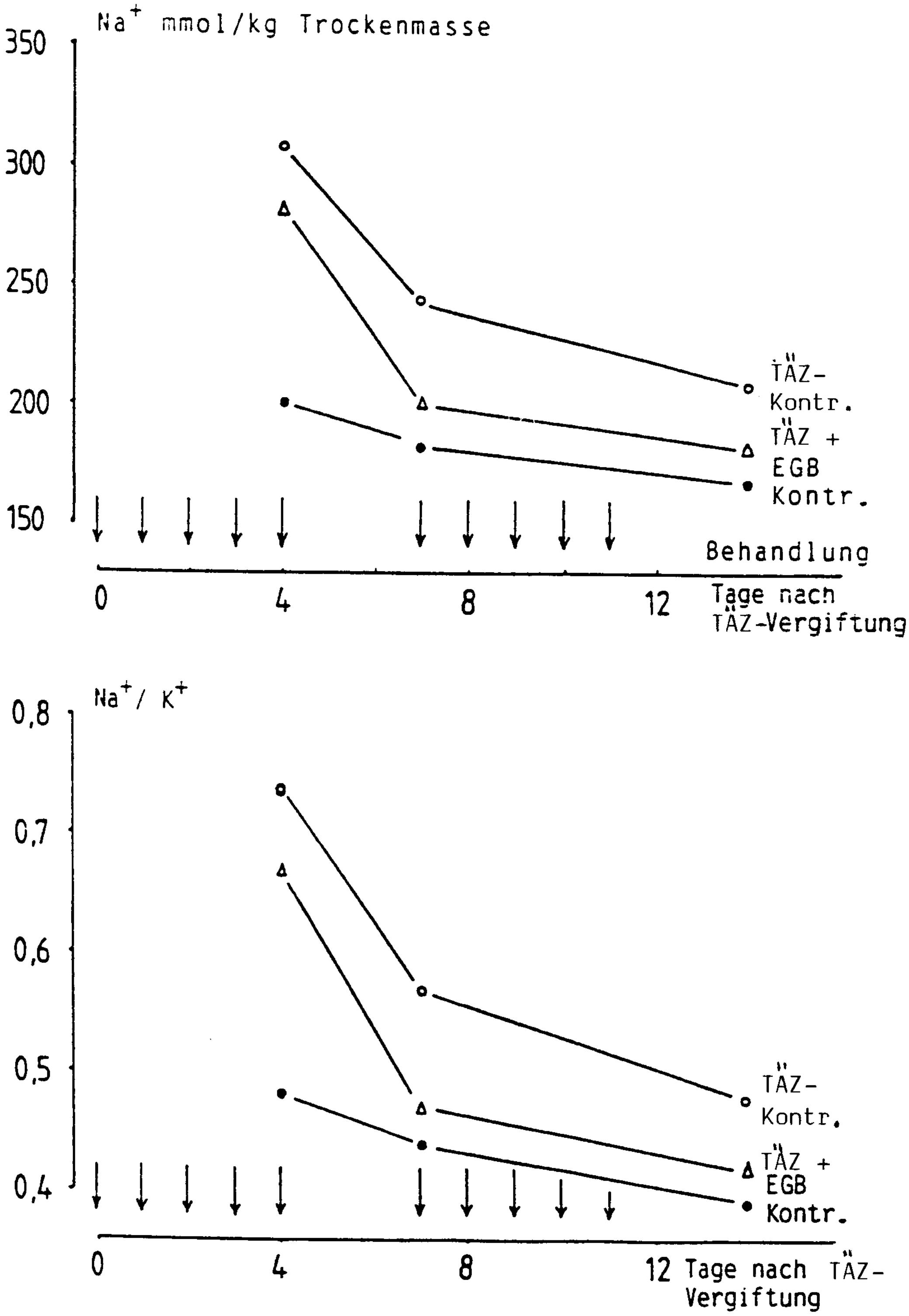
Na+ mmol/kg Trockenmasse
350
300
250
200
150
TÄZ-
Kontr.
TÄZ +
EGB
Kontr.
Behandlung
0
4
8
12
Tage nach
TÄZ-Vergiftung
Na+/ K+
0,8
0,7
0,6
0,5
0,4
TÄZ-
Kontr.
TÄZ +
EGB
Kontr.
0
4
8
12 Tage nach TÄZ-
Vergiftung

Abb. 2